# EUROPEAN PATENT APPLICATION

(11) **EP 4 567 100 A1**
(43) Date of publication of application: **11.06.2025**
(21) Application number: 23383260.9
(22) Date of filing: 05.12.2023
(51) Int. Cl.: C12N 5/071, G01N 33/50

(54) **ISOLATED HUMAN PORTAL VEIN ENDOTHELIAL CELL AND USES THEREOF**

(71) Applicant: Hospital Clínic de Barcelona, 08036 Barcelona (ES); Fundació de Recerca Clínic Barcelona-Institut d'Investigacions Biomèdiques August Pi I Sunyer, 08036 Barcelona (ES)
(72) Inventor: HERNÁNDEZ GEA, Virginia, 08950 ESPLUGUES DE LLOBREGAT (ES); SHALABY, Sarah, 08012 BARCELONA (ES); ANTON MARTÍNEZ, Aina, 08026 BARCELONA (ES); CAMPRECIÓS FIGUERAS, Genís, 08960 SANT JUST DESVERN (ES); GARCIA PAGÁN, Joan Carles, 08980 SANT FELIU DE LLOBREGAT (ES)
(74) Representative: ZBM Patents - Zea, Barlocci & Markvardsen

(57) **Abstract**

The present invention provides an isolated human portal vein endothelial cell (PVEC). The invention also provides an isolated cell population, a composition, and a microfluidic device comprising the isolated human PVEC. Further, the invention provides *in vitro* methods and uses of the isolated human PVEC, in particular for identifying a compound useful in the treatment and/or prevention of a hepatic disease or disorder. The invention also provides a *in vitro* method for preparing an isolated human PVEC.

## Description

### Technical Field

The present invention relates to the fields of cell biology and medicine. In particular, the present invention relates to isolated human portal vein endothelial cells (PVEC) and methods of obtaining and using such cells. The isolated human PVEC of the present invention are particularly useful for identifying drugs for preventing and treating portal hypertension.

### Background Art

The liver has a unique circulatory system evolved to protect against ischemic injury; 75% of blood inflow comes through the portal vein and the remaining 25% through the hepatic artery. The portal venous system (PVS) has several particularities when compared to the systemic venous system -it does not directly drain blood to the heart but into a high-resistance capillary network (hepatic sinusoid), lacks venous valves and does not have a pulsatile flow. Thus, in healthy conditions, PVS represents a unique vascular environment due to its low-pressure, slow flow and high-volume hemodynamics and the PV compliance is very high -the vessel can be easily distended to compensate variations in portal vein flow- making portal vein thrombosis (PVT) a rare event in the general population. However, PVT is a frequent and unresolved problem for patients with cirrhosis, with an incidence ranging from 10% to 26%.

PVT is generally associated with impaired liver function and worsening of portal hypertension (PH), the final driver of liver decompensation and liver-related mortality. Moreover, PVT adds technical complexity to the liver transplant (LT) procedure and may even prevent its feasibility in cases of extensive PVT. PVT also increases post-LT morbidity and mortality and increases the risk of developing PVT post-surgery, especially in the first days post-LT.

The pathophysiology of PVT is largely unknown, and by mirroring thrombosis from other venous territories, PVT is generally treated with anticoagulants. However, 30-60 % of PVT patients do not respond to anticoagulants, as opposed to the almost total response rate in thrombosis occurring in other vascular territories. This may be explained by the significant differences between the portal venous system (PVS) and the systemic venous system, as above explained.

Notably, it has remained greatly unexplored whether the portal vein endothelium plays any role in the pathophysiology of PVT, which may be explained by the vastly inaccessibility of the splanchnic territory and the absence of proper preclinical models. Animal models of venous thrombosis require venous wall damage and do not recapitulate the complexity of the cirrhosis-associated changes in the human splanchnic vascular bed. All these factors have hindered the development of effective treatments and accurate diagnostic or prognostic methods of PVT.

Thus, despite the significant research efforts carried out in the field during the last decades, there are no available research and clinical tools for studying and managing PVT, in particular, for identifying effective therapeutic agents against PVT.

### Summary of Invention

The present inventors, through a comprehensive study of the morphological and cellular changes that occur in the human portal vein during portal vein thrombosis (PVT), have surprisingly found that human portal vein endothelial cells (PVECs) play a central role in the development of PVT by causing portal vein wall thickening. Contrary to what may have been expected, the present inventors found that there is no deregulation of hemostatic pathways in the PVECs of PVT patients. Instead, they identified endothelial-to-mesenchymal transition (EndMT) as the key mechanism taking place in PVECs during cirrhosis with a maximal expression in patients with PVT. Thus, as shown in the examples below, the inventors unexpectedly found that, the main driver of PVT is not thrombus formation as in other thrombotic conditions of the venous territory, but rather endothelial wall thickening.

Additionally, the present inventors have successfully isolated, cultured, and immortalized human PVECs, and have demonstrated that they can be readily passaged and expanded *in vitro* with stable properties. As shown below, these isolated human PVECs have proved to represent an extraordinary useful research and clinical tool for assessing PVT. Notably, they have been used by the present inventors to identify new therapeutic treatments against PVT.

In conclusion, the present inventors have stablished a new research and clinical model -i.e., isolated human PVECs-, which is exceptionally useful for the study and clinical management of PVT, in particular for identifying new therapeutic treatments.

Thus, in a first aspect, the invention provides an isolated human portal vein endothelial cell (PVEC).

In a second aspect, the invention provides an isolated cell population comprising an isolated human portal vein endothelial cell (PVEC) as defined in the first aspect.

In a third aspect, the invention provides a composition comprising an isolated human portal vein endothelial cell (PVEC) as defined in the first aspect or an isolated cell population according to the second aspect.

In a fourth aspect, the invention provides a microfluidic device comprising an isolated human portal vein endothelial cell (PVEC) as defined in the first aspect, an isolated cell population as defined in the second aspect, or a composition as defined in the third aspect.

In a fifth aspect, the invention provides the *in vitro* use of an isolated human portal vein endothelial cell (PVEC) as defined in the first aspect, an isolated cell population as defined in the second aspect, a composition as defined in the third aspect, or a microfluidic device as defined in the fourth aspect, for identifying a compound useful in the treatment and/or prevention of a hepatic disease or disorder, particularly a compound useful in the treatment and/or prevention of portal vein thrombosis (PVT).

In a sixth aspect, the invention provides an *in vitro* method for identifying a compound useful for treating and/or preventing an hepatic disease or disorder, particularly portal vein thrombosis (PVT), the method comprising the step of contacting a compound with an isolated human portal vein endothelial cell (PVEC) as defined in the first aspect, an isolated cell population as defined in the second aspect, a composition as defined in the third aspect, or a microfluidic device as defined in the fourth aspect.

In a seventh aspect, the invention provides the *in vitro* use of an isolated human portal vein endothelial cell (PVEC) as defined in the first aspect, an isolated cell population as defined in the second aspect, a composition as defined in the third aspect, or a microfluidic device as defined in the fourth aspect, for diagnosing and/or prognosing a hepatic disease or disorder; particularly portal vein thrombosis (PVT); for deciding or recommending whether to initiate a medical regimen in a subject suspicious of suffering a hepatic disease or disorder, particularly portal vein thrombosis (PVT); or for determining the efficacy of a medical regimen in a subject already diagnosed with a hepatic disease or disorder, particularly portal vein thrombosis (PVT).

In an eighth aspect, the invention provides an *in vitro* method for diagnosing and/or prognosing a hepatic disease or disorder; particularly portal vein thrombosis (PVT); for deciding or recommending whether to initiate a medical regimen in a subject suspicious of suffering a hepatic disease or disorder, particularly portal vein thrombosis (PVT); or for determining the efficacy of a medical regimen in a subject already diagnosed with a hepatic disease or disorder, particularly portal vein thrombosis (PVT), the method comprising the step of contacting an isolated human portal vein endothelial cell (PVEC) as defined in the first aspect, an isolated cell population as defined in the second aspect, a composition as defined in the third aspect, or a microfluidic device as defined in the fourth aspect; with a sample from the subject.

In a nineth aspect, the invention provides an *in vitro* method for preparing an isolated human portal vein endothelial cell (PVEC), the method comprising the steps of a) dissociating the inner wall of a human portal vein or a part thereof to generate a cell suspension; b) plating the cell suspension onto a substrate which allows adherence of cells thereto; and c) culturing the plated cells under suitable conditions.

In a tenth aspect, the invention provides an isolated human portal vein endothelial cell (PVEC) obtainable by a method as defined in the tenth aspect.

In an eleventh aspect, the invention provides an isolated human portal vein endothelial cell (PVEC) as defined in the first or eleventh aspect, an isolated cell population as defined in the second aspect, a composition as defined in the third aspect, or a microfluidic device as defined in the fourth aspect, for use in medicine; particularly for the treatment and/or prevention of a hepatic disease or disorder, more particularly portal vein thrombosis (PVT).

In a twelfth aspect, the invention provides a compound useful for treating and/or preventing a hepatic disease or disorder, particularly portal vein thrombosis (PVT), identified by a method as defined in the sixth aspect.

### Brief Description of Drawings

Figure 1. Representative images of H&E staining of portal veins (A). Measurements of TI (tunica intima) and TM (tunica media) extrahepatic portal vein samples from polycystic liver disease (PLD) patients (normal portal vein) and cirrhotic patients with and without PVT (B).
Figure 2. Measurements of collagen, mucin and fibrin deposition in images of portal vein wall composition of PLD control patients, cirrhotic patients without PVT and cirrhotic patients with PVT, using Movat Pentachrome Stain Kit.
Figure 3. Scheme of PVEC obtention from a piece of PV tissue obtained during explant liver in a liver transplant.
Figure 4. Representative image of the mix of cells obtained after the isolation procedure from a human PV tissue. Non endothelial cells (triangle) mixed with endothelial cells (arrow) 6 days after cell isolation from a PV tissue.
Figure 5. Representative image of the phenotype at passage 1 of the PVEC. Uniform morphology, forming uniform monolayers.
Figure 6. Representative image of typical growing pattern seen in PVEC, forming small colonies and tight clusters.
Figure 7. Representative image non-endothelial cells.
Figure 8. Representative image of the senescence phenotype.
Figure 9. Endothelial cells becoming senescent and bigger (narrows).
Figure 10. Representative image of the immunofluorescence staining for typical endothelial markers: (A) eNOS and (B) vWF.
Figure 11. Tub formation assay. Representative image of the tub formation assay from PVEC.
Figure 12. Tub assay. Endothelial cells from the indicated different passages form tube structures when cultured in a Matrigel matrix.
Figure 13. Representative image of immortalized PVECs at passage 25.
Figure 14. Representative image of immortalized PVECs forming tubs in Matrigel matrix at passage 20.
Figure 15. FACS of immortalized PVECs showing that they conserve their endothelial markers (E-cadherin, y-axis; CD31, x-axis) at passage 22.
Figure 16. Representative image of immortalized PVECs showing that they did not form colonies in soft agar tumorigenic assay. PANC (epithelial cells from pancreatic cancer) did form colonies and serve as a positive control to show our PVECs and IM-PVECs did not acquire tumor features.
Figure 17. Fast Gene Set Enrichment Analysis (fGSEA) results from the comparison between CT_PVECs (control) and CH_PVECs (cirrhosis).
Figure 18. Fast Gene Set Enrichment Analysis (fGSEA) results from the comparison between CH_PVECs (cirrhosis) and CH_PVT_PVECs (cirrhosis with PVT).
Figure 19. Hallmark genes of EndMT. Gene expression levels of the indicated genes is presented in transcripts per million reads (tpm).

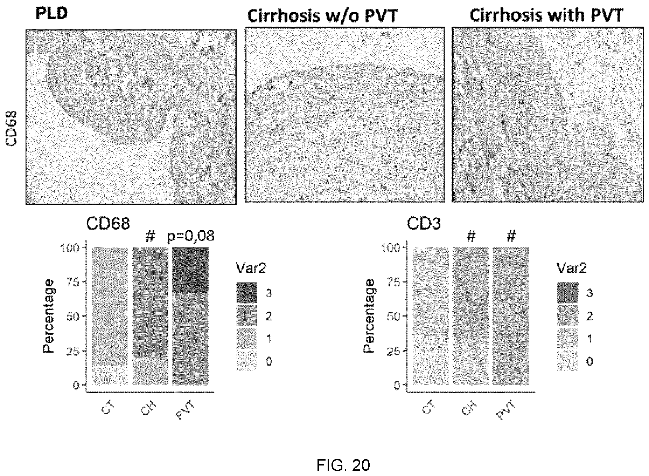

Figure 20. Representative images of CD68 staining of portal veins. Quantification of CD68 and Cd3 infiltration in the portal vein wall of PLD patients and cirrhotic patients with or without PVT.
Figure 21. PCR results from the in vitro treatment of PVECS with Simvastatin.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

As used herein, the indefinite articles "a" and "an" are synonymous with "at least one" or "one or more". Unless indicated otherwise, definite articles used herein, such as "the" also include the plural of the noun.

As above indicated, the present invention provides an isolated human portal vein endothelial cell (PVEC).

As used herein "portal vein endothelial cell" or "PVEC" refers to an endothelial cell that lines the inner walls of the hepatic portal vein. Endothelial cells can be identified by different methods well known in the art, for example, by measuring the expression of endothelial markers such as CD31, CD144, eNOS, and vWF. The term "isolated" as used herein refers to a cell or population of cells that have been removed from its *in vivo* location (e.g. liver tissue) and that is not associated with one or more cells or one or more cellular components with which the cell is associated *in vivo.* An isolated cell may have been removed from its native environment, or may result from propagation, e.g., *in vitro* propagation, of a cell that has been removed from its native environment. The term "*in vitro*" as used herein denotes outside, or external to, human body. The term "*in vitro*" as used herein should be understood to include "*ex vivo*"*.* The term "*ex vivo*" typically refers to tissues or cells removed from an animal or human body and maintained or propagated outside the body, e.g., in a culture vessel.

In a particular embodiment of the first aspect, the isolated human portal vein endothelial cell (PVEC) is originated, derived, obtained, or obtainable from human adult liver. In a more particular embodiment, the isolated human PVEC is originated, derived, obtained, or obtainable from the portal vein of human adult liver. The skilled person would understand that this is meant to also encompass cells that result from propagation of a cell that has been originated, derived, obtained, or obtained from human adult liver, in particular from the portal vein of human adult liver. "Adult liver" refers to liver of subjects that are any time after birth, preferably full term, and may be, e.g., at least 1 month, at least 2 months, at least 3 months, at least 4 months, at least 5 months, at least 6 months, at least 1 year, at least 5 years, at least 10 years, at least 15 years, at least 20 years, or at least 25 years of age after birth. Hence, an "adult liver", or mature liver, may be found in human subjects which would otherwise be described in the conventional terms of "infant", "child", "youth", "adolescent" or "adult".

In a more particular embodiment of the first aspect, the human adult liver is selected from the group consisting of human adult liver without cirrhosis and/or portal hypertension and cirrhotic human adult liver. In an even more particular embodiment, the cirrhotic human adult liver is a cirrhotic human adult liver without portal vein thrombosis (PVT) or a cirrhotic human adult liver with portal vein thrombosis (PVT). As shown below, the PVEC of cirrhotic livers or cirrhotic livers with PVT present alterations compared to PVEC from control subjects, in particular an endothelial mesenchymal transition (EndMT).

In one embodiment of the first aspect, the isolated human portal vein endothelial cell (PVEC) is a primary isolated human portal vein endothelial cell (PVEC).

In one embodiment of the first aspect, the isolated human portal vein endothelial cell (PVEC) is an *in vitro* cultured cell. A "cultured cell" refers to an isolated cell grown under controlled conditions.

In one embodiment of the first aspect, the isolated human portal vein endothelial cell (PVEC) expresses at least one endothelial cell surface marker. In another embodiment, the isolated human portal vein endothelial cell (PVEC) expresses the cell surface marker CD31 (cluster of differentiation 31) and/or CD144 (cluster of differentiation 144). In a more particular embodiment, the isolated human PVEC further expresses the cell surface marker eNOS (Endothelial Nitric Oxide Synthase) and/or vWF (von Willebrand factor). In an even more particular embodiment, the isolated human PVEC expresses at least one cell surface marker selected from the group consisting of CD31, CD144, eNOS, vWF, and combinations thereof. In an even more particular embodiment, the isolated human PVEC expresses the cell surface markers CD31, CD144, eNOS, and vWF. In a particular embodiment, the cell surface markers are determined by flow cytometry and/or immunofluorescence. In a more particular embodiment, the cell surface markers CD31 and CD144 are determined by flow cytometry and the cell surface markers eNOS and vWF are determined by immunofluorescence.

In another embodiment, the isolated human portal vein endothelial cell (PVEC) comprises an enhanced endothelial mesenchymal transition (EndMT); particularly in comparison with a control endothelial cell. As below explained, methods and markers to determine the EndMT of cells are well known in the art.

In a more particular embodiment, the isolated human PVEC comprises at least one of:
- increased expression of at least one mesenchymal marker; particularly wherein the mesenchymal marker is selected from the group consisting of N-cadherin (CDH2), FSP-1 (AIFM2), LOXL2, and combinations thereof;
- increased expression of at least one adhesion marker, particularly VCAM1;
- increased expression of SNAI1 and/or ZEB2;
- increased expression of RAC2 and/or SERPINE1; and
- increased expression of COL13A1 and/or MMP16;
particularly in comparison with a control endothelial cell.

In an even more particular embodiment, the isolated human PVEC comprises:
- increased expression of at least one mesenchymal marker; particularly wherein the mesenchymal marker is selected from the group consisting of N-cadherin (CDH2), FSP-1 (AIFM2), LOXL2, and combinations thereof;
- increased expression of at least one adhesion marker, particularly VCAM1;
- increased expression of SNAI1 and/or ZEB2;
- increased expression of RAC2 and/or SERPINE1; and
- increased expression of COL13A1 and/or MMP16;
particularly in comparison with a control endothelial cell.

Wherein a cell is said to express a particular marker, or an increased expression of a particular marker, this means that a skilled person will conclude the presence or evidence of a distinct signal for that marker when carrying out the appropriate measurement, compared to suitable controls. Where the method allows for quantitative assessment of the marker, positive cells may on average generate a signal that is significantly different from the control, e.g., but without limitation, at least 1-fold, at least 1.5-fold, at least 2-fold, at least 4-fold, at least 10-fold, at least 20-fold, at least 30-fold, at least 40-fold, at least 50-fold higher or even higher, than such signal generated by control cells. The expression of cell-specific markers, in particular surface markers, can be detected using any suitable immunological technique known in the art, such as flow cytometry, immuno-cytochemistry (i.e., immunofluorescence) or affinity adsorption, Western blot analysis, ELISA, etc., or by any suitable technique of measuring the quantity of the marker mRNA, e.g., Northern blot, semi-quantitative or quantitative RT-PCR, etc. By means of illustration and not limitation, cells displaying one or more surface molecules characteristic of the PVEC cells of the invention, e.g., one or more of the markers listed above, may be recognized by specific (labelled) antibodies or other recognition agents against such as markers and sorted out from cells not displaying such surface molecules, e.g., using fluorescence activated cell sorting or using affinity binding to, e.g., columns, beads or surfaces (panning). Any other ways of enrichment for the cells are also included in the invention.

In one embodiment of the first aspect, the isolated human portal vein endothelial cell (PVEC) comprises a cell surface marker expression profile that remains substantially unchanged with passage or origin.

In one embodiment of the first aspect, the isolated human portal vein endothelial cell (PVEC) is capable of forming tubes when cultured in Matrigel. The conditions for culturing the cells in Matrigel and determining the formation of tubes are well known in the art and can be, for example, the conditions used in the examples below.

In one embodiment of the first aspect, the isolated human portal vein endothelial cell (PVEC) is capable of undergoing at least 5, at least 6, at least 7, at least 8, or at least 9, passages in culture. In another embodiment, the isolated human portal vein endothelial cell (PVEC) is capable of undergoing up to 7, up to 8, or up to 9 passages in culture. As used herein, a "passage" refers to each round of cell subculturing. It will be understood by those of skill in the art that there may be many population doublings during the period of passaging; therefore the number of population doublings of a culture is usually greater than the passage number. The expansion of cells (i.e., the number of population doublings) during the period between passaging depends on many factors, including, but not limited to the seeding density, substrate, medium, and time between passaging.

The isolated human PVEC of the invention can be modified, e.g., genetically modified, for instance to increase their replicative capacity (i.e., to immortalize them). Any methods of immortalizing cells are contemplated in the present application, such as genetically altering the cells with DNA encoding the SV40 large T antigen (for example, as described in WO1997/32972), infecting with Epstein Bar Virus, introducing oncogenes such as myc and/or ras, introducing viral replication genes such as adenovirus E1a, telomerization, and fusing cells having the desired phenotype with an immortalized cell line. As used herein, "immortalized cell" refers to a cell where the limitation of the number of cell divisions by the Hayflick limit has been abrogated or reduced. Thus, immortalized cells may divide any number of times, or at least a significantly higher number of times compared to the corresponding primary cells. Immortalized cells generally retain the self-function of primary cells from which the derive.

In one embodiment of the first aspect, the isolated human portal vein endothelial cell (PVEC) is an immortalized cell. That is, an isolated human PVEC that has been immortalized. In a more particular embodiment, the immortalized cell comprises or expresses an SV40 antigen, optionally under the control of a heterologous promoter. In an even more particular embodiment, the immortalized cell comprises or expresses as SV40 large T-antigen, optionally under the control of a heterologous promoter.

In one embodiment of the first aspect, the isolated human portal vein endothelial cell (PVEC) is an immortalized cell that can undergo at least 10, at least 15, at least 20, at least 25, at least 30, at least 35, or at least 40 passages in culture. In another embodiment, the immortalized cell can undergo up to 25, up to 30, up to 35, or up to 40 passages in culture.

In one embodiment of the first aspect, the isolated human portal vein endothelial cell (PVEC) is an immortalized cell that is non-tumorigenic (i.e., does not form colonies when culture in soft agar).

The isolated human PVEC of the invention can be engineered using any of a variety of vectors including, but not limited to, integrating viral vectors, e.g., retroviral vector, adeno-associated viral vector, lentiviral vector; non-integrating replicating vector, e.g., papilloma virus vectors, SV40 vectors, adenoviral vectors; or replication-defective viral vectors. Other methods of introducing DNA into cells include the use of liposomes, electroporation, a particle gun, or by direct DNA injection.

The human isolated PVEC of the invention can be obtained, as explained in the examples below, from liver samples, for example obtained during liver transplant.

As above indicated, in a second aspect the invention provides an isolated cell population comprising an isolated human portal vein endothelial cell (PVEC) as defined in the first aspect. All the embodiments above provided for the first aspect are also meant to apply to this second aspect.

In one embodiment of the second aspect, the cell population is a cell line.

The term "cell population" refers generally to a grouping of cells, that can be homogeneous or heterogeneous. The term "cell line" refers to a population of largely or substantially identical cells that has typically been derived from a single ancestor cell or from a defined and/or substantially identical population of ancestor cells. The cell line may be capable of being maintained in culture for an extended period (e.g., months, years, for an unlimited period of time).

A cell population may consist of cells having a common phenotype or may comprise at least a fraction of cells having a common phenotype. Cells are said to have a common phenotype when they are substantially similar or identical in one or more demonstrable characteristics, including but not limited to morphological appearance, the presence, absence or level of expression of particular cellular components or products, e.g., RNA, proteins or other substances, activity of certain biochemical pathways, surface markers, proliferation capacity and/or kinetics, differentiation potential and/or response to differentiation signals or behaviour during in vitro cultivation (e.g., adherence, non-adherence, monolayer growth, proliferation kinetics, or the like). Such demonstrable characteristics may therefore define a cell population or a fraction thereof.

In one embodiment of the second aspect, at least 50%, at least 60%, at least 70%, at least 80%, at least 90%, at least 95%, or at least 99% of the cells in the cell population are isolated human PVEC as defined in the first aspect. In a more particular embodiment, all the cells in the cell population are isolated human PVEC as defined in the first aspect.

As above indicated, in a third embodiment the invention provides a composition comprising an isolated human portal vein endothelial cell (PVEC) as defined in the first aspect or an isolated cell population according to the second aspect.

In one embodiment of the third aspect, the composition is a pharmaceutical composition comprising therapeutically effective amount of the isolated human PVEC or the isolated cell population together with at least one pharmaceutically acceptable excipient, diluent or carrier.

The expression "pharmaceutical composition" encompasses both compositions intended for human as well as for non-human animals. The skilled in the art understands that a pharmaceutical composition must comprise a therapeutically effective amount of the active compound. The expression "therapeutically effective amount" as used herein, refers to the amount of the cell or cell population that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipient, diluent or carrier" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio.

As above indicated, in a fourth aspect the invention provides a microfluidic device.

The term "microfluidic device", as used herein, refers to a device having features of micron or submicron dimensions, and which can be used in any number of processes involving very small amounts of fluid. Such processes include, but are not limited to, screening and diagnostics The features of the microfluidic devices can be adapted to their particular use by the skilled person without the need of any inventive skill.

In one embodiment, the microfluidic device comprises a permanent or degradable decellularized or synthetic matrix or scaffold. In a particular embodiment, the microfluidic device comprises a hydrogel. In a more particular embodiment, the hydrogel is a biocompatible hydrogel. As used herein, "hydrogel" or "hydrogel matrix" are used interchangeably and refer to a substance formed by a proteins or protein fragments crosslinked via covalent, ionic, or hydrogen bonds to create a three-dimensional open-lattice structure which entraps water molecules to form a gel. "Biocompatible hydrogel" refers to a polymer that forms a gel which is not toxic to living cells and allows sufficient diffusion of oxygen and nutrients to the entrapped cells to maintain viability.

In a particular embodiment, the hydrogel comprises proteins or protein fragments, and optionally a cross-linking agent. In a more particular embodiment, the hydrogel comprises collagen, and optionally a cross-linking agent. In an even more particular embodiment, the hydrogel comprises gelatin (i.e., collagen hydrolysate), particularly gelatin methacryloyl. In another particular embodiment, the hydrogel comprises gelatin, alginate, and fibrinogen. A "protein" as used herein, refers to a polymer made up of amino acids. This term is meant to include proteins, polypeptides, peptides, or fragments thereof, wherein the proteins, polypeptides, or peptides are natural or synthetic. For example, in some embodiments, the protein is collagen. Exemplary proteins herein are any that are capable of transitioning from liquid solution to a hydrogel. The transition generally can occur spontaneously as a function of time, temperature, concentration of protein, and other factors. The term "cross-linking agent" refers to a monomer containing at least two reactive groups capable of forming covalent linkages with the protein that forms the hydrogel. As used herein, the term "collagen" refers to a family of homotrimeric and heterotrimeric proteins comprised of collagen monomers. Any collagen can be used to generate the microfluidic devise of the invention. The term "collagen hydrolysate" and "gelatin" are used interchangeably and refer to compositions comprising collagen fragments. The collagen monomers may be fibrillar collagen monomers or non-fibrillar collagen monomers. Collagen hydrolysates are commonly formed by acid or basic hydrolysis of collagen.

In another embodiment of the fourth aspect, the microfluidic device comprises one or more internal spaces in the hydrogel matrix, optionally coated with the isolated human portal vein endothelial cell (PVEC) or the isolated cell population. In a more particular embodiment, the internal spaces are microchannels.

In a particular embodiment of the fourth aspect, the microfluidic device further comprises a human fluid sample. In a more particular embodiment, the human fluid sample is human blood or serum. The human fluid sample, in particular the human blood or serum, may be derived, without limitation, from a healthy subject, a cirrhotic patient, or a cirrhotic patient with PVT.

As above indicated, in a fifth and sixth aspects the invention provides an *in vitro* use and method for identifying a compound useful in the treatment and/or prevention of a hepatic disease or disorder, particularly useful in the treatment and/or prevention of portal vein thrombosis (PVT).

The term "treatment", as used herein, unless otherwise indicated, refers to any type of therapy which is aimed at terminating, preventing, ameliorating or reducing the susceptibility to a clinical condition or existing disease as described herein, including complete curing of a disease as well as amelioration or alleviation of said disease. Thus, "treatment," "treating," and their equivalent terms refer to obtaining a desired pharmacologic or physiologic effect, covering any treatment of a pathological condition or disorder in a subject. The effect may be prophylactic in terms of completely or partially preventing a disorder or symptom thereof and/or may be therapeutic in terms of a partial or complete cure for a disorder and/or adverse effect attributable to the disorder. That is, "treatment" includes preventing the disorder from occurring or recurring in a subject, inhibiting the disorder, such as arresting its development, stopping or terminating the disorder or, at least, clinical signs associated therewith, so that the host no longer suffers from the disorder or its clinical signs, such as causing regression of the disorder or its clinical signs, for example, by restoring or repairing a lost, missing or defective function, or stimulating an inefficient process, or relieving, alleviating, or ameliorating the disorder, or clinical signs associated therewith, where ameliorating is used in a broad sense to refer to at least a reduction in the magnitude of a clinical sign parameter. The term "prevention" or "preventing", as used herein, means, but is not limited to, a process of prophylaxis in which a subject is exposed to the fusion protein of the invention prior to the induction or onset of the disease process. Altogether, such treatment results in prevention or reduction of the clinical signs of the disease or disorder. A "disease" is a state of health wherein the subject cannot maintain homeostasis, and wherein if the disease is not ameliorated then the subject's health continues to deteriorate. In contrast, a "disorder" is a state of health in which the subject is able to maintain homeostasis, but in which the subject's state of health is less favorable than it would be in the absence of the disorder. Left untreated, a disorder does not necessarily cause a further decrease in the subject's state of health.

In a particular embodiment of the fifth and sixth aspects, the compound is determined to be useful in the treatment and/or prevention of a hepatic disease or disorder, particularly useful in the treatment and/or prevention of portal vein thrombosis (PVT), when the compound causes a change in phenotype in the isolated human PVEC, for example, and without limitation, a change in cell survival, morphology, functional or physiological characteristic, and/or a molecular marker. In a more particular embodiment, the compound is determined to be useful in the treatment and/or prevention of a hepatic disease or disorder, particularly useful in the treatment and/or prevention of portal vein thrombosis (PVT), when the compound reduces, prevents, or reverts the endothelial-to-mesenchymal transition (EndMT) of the isolated human PVEC. In a more particular embodiment, the compound is determined to be useful in the treatment and/or prevention of portal vein thrombosis (PVT) when the compound reduces, prevents, or reverts the endothelial-to-mesenchymal transition (EndMT) of an isolated human PVEC originated, derived, obtained, or obtainable from a cirrhotic human adult liver without portal vein thrombosis (PVT). In a more particular embodiment, the compound is determined to be useful in the treatment and/or prevention of portal vein thrombosis (PVT) when the compound reduces, prevents, or reverts the endothelial-to-mesenchymal transition (EndMT) of an isolated human PVEC originated, derived, obtained, or obtainable from a cirrhotic human adult liver with portal vein thrombosis (PVT). Methods to determine the EndMT of cells are well known in the art and can be, for example, those disclosed in the example below which are based on the determination of hallmarks of EndMT (e.g., the loss of EC markers and functions PECAM1 and TIE1, and the gain in mesenchymal cell markers and functions N-CADH and FSP-1 and LOXL2).

In a particular embodiment of the sixth aspect, the method further comprises the step of determining a change in phenotype in the isolated human PVEC or isolated cell population. In a more particular embodiment, the method further comprises the step of determining the endothelial-to-mesenchymal transition (EndMT) of the isolated human PVEC or isolated cell population. In an even more particular embodiment, the method further comprises the step of determining the endothelial-to-mesenchymal transition (EndMT) of the isolated human PVEC or isolated cell population originated, derived, obtained, or obtainable from a cirrhotic human adult liver with or without portal vein thrombosis (PVT).

In one embodiment of the seventh and eighth aspects, the medical regimen is anticoagulation therapy. As used herein, "anticoagulation therapy" or "anticoagulant" refers to an agent or class of agents that prevents clotting or clotting of blood, such as heparin.

In one embodiment of the eighth aspect, the method further comprises the step of determining a change in phenotype in the isolated human PVEC. In a more particular embodiment, the method further comprises the step of determining the endothelial-to-mesenchymal transition (EndMT) of the isolated human PVEC. In a more particular embodiment, the method further comprises the step of comparting the EndMT of the isolated human PVEC with a reference value.

In a particular embodiment of the fifth to eighth aspects, the isolated human PVEC is originated, derived, obtained, or obtainable from a human adult liver, optionally selected from the group consisting of human adult liver without liver cirrhosis and/or portal hypertension, and cirrhotic human adult liver. In another embodiment, the human adult liver is selected from the group consisting of human adult liver without liver cirrhosis and/or PVT, and cirrhotic human adult liver. In another embodiment, the human adult liver is healthy human adult liver. In an even more particular embodiment, the cirrhotic human adult liver is a cirrhotic human adult liver without portal vein thrombosis (PVT) or cirrhotic human adult liver with portal vein thrombosis (PVT). In one embodiment, the isolated human PVEC is originated, derived, obtained, or obtainable from a cirrhotic human adult liver with portal vein thrombosis (PVT).

In a particular embodiment of the fifth, sixth, seventh, eighth, and eleventh aspects, the hepatic disease or disorder is splanchnic vascular dysfunction or vascular liver disease. In a more particular embodiment, the vascular liver disease is selected from the group consisting of cirrhosis, porto-sinusoidal vascular disease (PSVD), portal hypertension, portal vein thrombosis (particularly, portal vein thrombosis in liver cirrhosis), and portal cholangiopathy.

In a particular embodiment of the seventh and eighth aspects, a subject suspicious of suffering a hepatic disease or disorder, particularly portal vein thrombosis (PVT), is a subject (i.e., a patient) not responding to anticoagulant treatment.

In a particular embodiment of the eighth aspect, the sample is a fluid human sample, particularly human blood or serum.

As above indicated, in a nineth aspect the invention provides an *in vitro* or ex *vivo* method for preparing an isolated human portal vein endothelial cell (PVEC). All embodiment above disclosed for the isolated human PVEC of the first aspect are also meant to apply to this aspect. Thus, in one embodiment of the nineth aspect, the isolated human PVEC is as defined in the first aspect.

The term "disassociating" as used herein generally refers to partly or completely disrupting the cellular organization of a tissue or organ, i.e., partly or completely disrupting the association between cells and cellular components of a tissue or organ. As can be understood by a skilled person, the aim of disassociating a tissue or organ is to obtain a suspension of cells from the tissue or organ. The suspension may comprise solitary or single cells, as well as cells physically attached to form clusters or clumps of two or more cells. Disassociating preferably does not cause or causes as small as possible reduction in cell viability.

A suitable method for disassociating human portal vein may be any method well known in the art, including but not limited to, enzymatic digestion, mechanical separation, filtration, centrifugation and combinations thereof. In one embodiment, the dissociating in step a) is carried out by enzymatic treatment and/or mechanical treatment. In a particular embodiment, the enzymatic treatment comprises contacting the inner wall of the human portal vein or a part thereof with a protease. In another embodiment, the enzymatic treatment comprises opening the human portal vein or a part thereof and contacting the inner wall of the human portal vein or a part thereof with a protease. Any protease suitable to dissociated tissues is encompassed by the present invention. In a more particular embodiment, the enzymatic treatment comprises contacting the inner wall of the human portal vein or a part thereof with trypsin. In another embodiment, the enzyme, particularly trypsin, is contacted until at least a portion of the human portal vein is dissociated into individual cells. In a more particular embodiment, the enzyme, particularly trypsin, is contacted for at least 1 min, at least 2 min, at least 3 min, at least 4 min, or at least 5 min, optionally at 37 °C. In another particular embodiment, the trypsin is contacted from 1 min to 15 min, from 2 to 10 min, from 3 to 8 min, or from 4 to 6 min, optionally at 37 °C. The skilled person would know how to adjust the conditions of enzymatic treatment for obtaining optimal results by routine experimentation. In another particular embodiment, the mechanical treatment comprises scraping the inner wall of the human portal vein, optionally in culture medium. In an even more particular embodiment, the dissociating in step a) is carried out, in the following order, by an enzymatic treatment and a mechanical treatment. In another particular embodiment, the dissociating in step a) is carried out, optionally in the following order, by contacting the inner wall of the human portal vein or a part thereof with a protease, particularly trypsin, and scraping the inner wall of the human portal vein.

The term "plating" as used herein is synonymous to seeding or inoculating, and in general refers to introducing a cell or cell population into an *in vitro* environment capable of promoting the survival and/or growth of the introduced cells. Typically, the said environment may be provided in a system which is suitably delimited from the surroundings, such that it can prevent an undesired exchange of matter between the said environment and the surroundings (thereby avoiding, e.g., contamination of the environment or escape of culture medium or cells therefrom), while it can allow for continuous or intermittent exchange of other, useful, matter components between the said environment and the surroundings (e.g., an occasional exchange of a part or all of the culture medium, the continuous exchange of gases, or the harvesting of the cells after culturing, etc.). Usually, environments suitable for culturing of cell can be generated in culture vessels well-known in the art, such as, e.g., cell culture flasks, well plates and dishes of various formats. In the present invention, cells are plated onto a substrate which allows for adherence of cells thereto, i.e., a surface which is not generally repulsive to cell adhesion or attachment. This may be carried out, e.g., by plating the cells in a culture system (e.g., a culture vessel) which displays one or more substrate surfaces compatible with cell adhesion. When the said one or more substrate surfaces contact the suspension of cells (e.g., suspension in a medium) introduced into the culture system, cell adhesion between the cells and the substrate surfaces may ensue. Accordingly, the term "plating onto a substrate which allows adherence of cells thereto" refers to introducing cells into a culture system which features at least one substrate surface that is generally compatible with adherence of cells thereto, such that the plated cells can contact the said substrate surface. General principles of maintaining adherent cell cultures are well-known in the art. In general, a substrate which allows adherence of cells thereto may be any substantially hydrophilic substrate. As known in the art, culture vessels, e.g., culture flasks, well plates, dishes, or the like, may be usually made of a large variety of polymeric materials, including, but not limited to polyacrylates, polymethylacrylates, polycarbonates, polystyrenes, polysulphones, polyhydroxyacids, polyanhydrides, polyorthoesters, polyphosphazenes, polyphosphates, polyesters, nylons or mixtures thereof, etc. Generally, culture vessels made of such materials are surface treated after moulding in order to provide for hydrophilic substrate surfaces and thereby enhance the likelihood of effective cell attachment. Surface treatment may take the form of a surface coating, or may involve the use of directed energy at the surface with the intention of generating chemical groups on the polymer surface. These chemical groups will have a general affinity for water or otherwise exhibit sufficient polarity to permit stable adsorption to another polar group. These functional groups lead to hydrophilicity and or an increase in surface oxygen and are properties recognized to enhance cell growth on so modified substrate surfaces. Such chemical groups may include groups such as amines, amides, carbonyls, carboxylates, esters, hydroxyls, sulfhydryls and the like. Current standard practices for growing adherent cells, in particular endothelial cells, may involve the use of defined chemical media with addition of bovine, human or other animal serum. The added serum, besides providing nutrients and/or growth promoters, may also promote cell adhesion by coating the treated plastic surfaces with a layer of matrix to which cells can better adhere. An alternative substrate surface compatible with cell adhesion may be glass, optionally surface treated to introduce functional groups, e.g., as listed above, to increase the hydrophilicity thereof. Other adherent substrate surfaces may be generated via surface coating, e.g., coating of the polymeric or treated polymeric surfaces as above. In a non-limiting example, the coating may involve suitable poly-cations, such as, e.g., poly-omithine or poly-lysine. In a particular embodiment, the substrate is coated with human plasma fibronectin. In a more particular embodiment, the substrate is coated with human plasma fibronectin at concentration from 0.1 µg/cm² to 2 µg/cm², 0.5 µg/cm² to 1.5 µg/cm², or of about 1 µg/cm²

The skilled person would know what are the suitable conditions for culturing cells as they belong to the common general knowledge. In a particular embodiment, the culturing in step c) is carried out at atmospheric conditions comprising about 5% CO₂, a temperature from 35°C to 39°C, particularly at 37°C, and/or a relative humidity of about 100%.

In one embodiment of the eleventh aspect, the culturing in step c) is carried out in endothelial growth medium (EGM). There are several EGM well known in the art and also commercially available. For example, EGM^{™}-2 Endothelial Cell Growth Medium-2 BulletKit^{™} (EGM-2) (Cultek, #CC-3162). In another embodiment, the step c) is carried out until confluence is reached. In another embodiment, step c) is carried out for at least 1 day, at least 2 days, at least 3 days, at least 4 days, at least 5 days, at least 6 days, or at least 7 days.

In another embodiment of the nineth aspect, the method further comprises, after step a) and before step b), the step a') centrifuging the cell suspension to generate a cell pellet and a supernatant, discarding the supernatant, and resuspending the cell pellet in endothelial growth medium to form a second cell suspension. The skilled person would know how to adjust the centrifugation conditions to obtain optimal results. For example, cells can be centrifuged at 300 g for 5 min as shown in the examples below.

In one embodiment of the nineth aspect, the method further comprises the step d) isolating the cells that express at least one endothelial surface marker. In another embodiment of the nineth aspect, the at least one endothelial surface marker is selected from the group consisting of CD31, CD144, eNOS, vWF, and combinations thereof. In a more particular embodiment, the method further comprises isolating the cells that at least express the surface markers CD31 and CD144, in particular the surface markers CD31, CD144, eNOS, and vWF. This isolating step can be carried out with any standard technique known by the skilled in the art, such as those disclosed in the examples below.

In a tenth aspect, the invention provides an isolated human portal vein endothelial cell (PVEC) obtainable by a method as defined in the nineth aspect.

The isolated human PVEC "obtainable by" the method as defined above is used herein to define the isolated human PVEC by its preparation process and relates to the isolated human PVEC obtainable by the preparation method which comprises the steps described above. For the purposes of the invention, the expressions "obtainable", "obtained" and equivalent expressions are interchangeably used, and in any case the expression "obtainable" includes the expression "obtained".

The embodiments mentioned above regarding the method of the nineth aspect also apply to the isolated human PVEC obtainable by its preparation process.

As above indicated in an eleventh aspect the invention provides the isolated human PVEC, the cell population, the composition, or the microfluidic device of the invention, for use in medicine, particularly for the diagnosis, prognosis, or treatment of a hepatic disease or disorder, in particular portal vein thrombosis (PVC).

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

Abbreviations: Portal Vein (PV) , extracellular matrix (ECM), portal vein thrombosis (PVT), hepatocellular carcinoma (HCC), polycystic liver disease (PLD), hepatitis C virus (HCV), hepatitis B virus (HBV), hepatitis D virus (HDV), human immunodeficiency virus (HIV), magnetic resonance imaging (MRI), Hematoxylin and eosin (H&E), portal vein endothelial cells (PVECs), hepatic vein endothelial cells (HVECs), hepatic vein control patients (HV_CT), control patients with PLD but without signs of portal hypertension (P_CT), cirrhotic patients without PVT (P_CH), cirrhotic patients with PVT (P_PVT), differentially expressed genes (DEGs), transcripts per million (TPM), Drug Gene Interaction Database (DGldb), Short Time-course Expression Miner (STEM), endothelial to mesenchymal transition (EndMT), quantitative real-time polymerase chain reaction (qPCR), fast gene set enrichment analysis (fGSEA), transcription factors (TF), tunica intima (Ti), tunica media (TM).

### Materials and methods

### Isolation and immortalization of portal vein endothelial cells (PVECs)

Portal vein tissues were prospectively collected from explanted livers right after liver hepatectomy of cirrhotic patients with (n =5) or without portal vein thrombosis (PVT) (n=12), regardless of the underlying etiology or the presence of hepatocellular carcinoma (HCC), as well as from explanted livers of polycystic liver disease (PLD) patients without signs of portal hypertension (n=3) undergoing orthotopic liver transplantation.

Exclusion criteria of cirrhotic patients were the presence of PVT due to HCC macrovascular invasion and the patient presentation of active HCV, HBV, HDV or HIV viruses at the time of surgery. PLD patients presenting portal hypertension were also excluded from the study. Tissues were rapidly immersed in EGM^{™}-2 Endothelial Cell Growth Medium-2 BulletKit^{™} (EGM-2) (Cultek, #CC-3162) between 10 and 60 minutes after hepatectomy and samples were processed between 1 and 6 h after collection.

Patients were divided in two groups: portal vein thrombosis (PVT) and no PVT. PVT was diagnosed by demonstrating presence of endoluminal material compatible with non-tumoral thrombosis in the portal vein and/or its branches at Doppler-US. PVT diagnosis and its extension were always confirmed by computed tomography (CT) or magnetic resonance imaging (MRI) and classified according to the latest expert consensus (de Franchis, Roberto et al. "Baveno VII - Renewing consensus in portal hypertension." Journal of hepatology vol. 76,4 (2022): 959-974).

Occasionally, PVT was discovered intraoperatively during the course of liver transplantation. Patients classified as PVT included those who had a recent history of PVT but with no active thrombi at the time of liver transplantation (recanalization) and those who arrived at surgery with an existing thrombus.

### Endothelial cells isolation from human portal vein

For endothelia cell (EC) isolation from vein tissue -either portal vein for obtaining PVEC or hepatic vein for obtaining HVEC- excess surrounding fat tissue was meticulously removed from the vein using sterile scissors, and any residual blood was washed away with DPBS. Subsequently, the tubular vein was opened to expose the inner part of the vein facing upward. Trypsin EDTA 0,05% (Gibco, #25300054) was then added on the upper surface and incubated for 5 minutes at 37°C. EGM-2 medium was then added abundantly over the tissue and a cell scrapper (TPP-Reactiva, #99002) was used to carefully scrap the surface of the inner wall several times, in order to detach the endothelial cells from the vein wall. The EGM-2 medium with cells in suspension was then centrifuged at 300 g for 5 minutes and pellet resuspended in 2 ml of fresh EGM-2. Cells were then seeded in a well from a 6-well plate pre-coated with human plasma fibronectin at a concentration of 1 µg/cm² (Sigma, #F0895), and fresh media changed every other day until confluence. On average, confluence was reached 10 days after isolation.

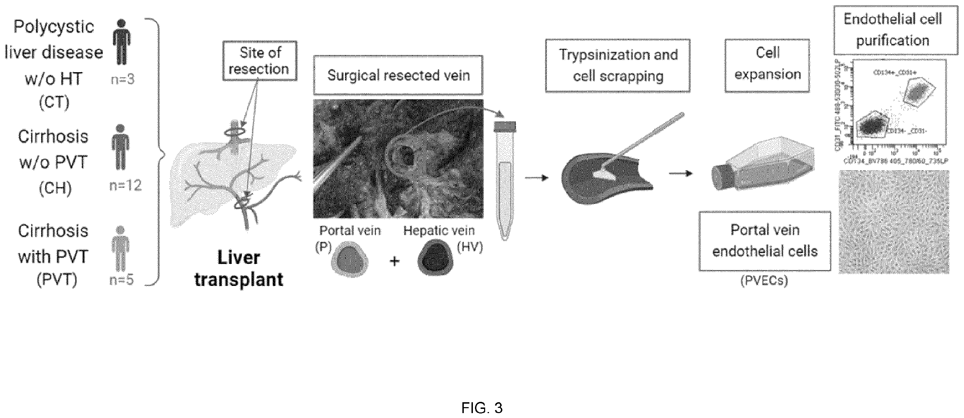

In order to isolate ECs from other cell types that were also scrapped during the initial tissue processing, cells were detached, stained with anti-CD31-FITC (BD Bioscience, #555445) and CD144 -BV786 (BD Bioscience, #565672) antibodies and FACS sorted in a FACS Aria II cell sorter (BD Biosciences) as explained below. ECs were selected as CD31+ CD144⁺ cells and seeded back to either a 6-well plate, T25 or T75 flasks, depending on the cell amount obtained (Figure 3). Once ECs reached confluency, a third of the cells were collected in RLT buffer (Qiagen) for subsequent RNA isolation (passage 1), a third was used for flow cytometry purity analysis and the final one third was passaged into a new 6-well plate. From this moment onwards, both portal vein endothelial cells (PVECs) and hepatic vein endothelial cells (HVECs) were expanded at a split ratio of 1:5 until senescence. At every passage, a fraction of the cells was cryopreserved for future experiments, while the rest was used for flow cytometry purity check, RLT collection and tube assays.

Endothelial phenotype of PVECs and HVECs was routinely monitored by cell morphology, flow cytometry, immunofluorescence and tube formation capacity.

### Flow Cytometry and FACS sorting

Endothelial markers expression of CD31 and CD144 were analyzed by flow cytometry at every passage. Briefly, cells were washed, trypsinized with 0.05 mg/mL trypsin-EDTA solution for 5 min, and centrifuged at 300 ×g for 5 min. Cells were then re-suspended in 100 µl of PBS + 5% FBS (staining buffer) and unspecific binding blocked with 10% goat serum (Dako, #X0907) for 10 min at 4°C. Cells were then stained with 1µl of CD31-FITC (BD Bioscience, #555445) and 1µl of CD144-BV786 (BD Bioscience, #565672) antibodies on the dark for 20 min at 4°C. Antibodies were washed by adding 1 ml of staining buffer and centrifuged 300 xg for 5 min. Cells were finally resuspended in 200 µl of staining buffer containing 1 µg/mL DAPI (4',6-diamidino-2-phenylindole, ThermoFisher, #D1306) and either analyzed in a FACS Canto III flow cytometer (BD Bioscience) or ECs sorted in a FACS Aria II cell sorter, as stated above. Data were analyzed with the Flowjo 10.0 software.

### Tub assay

The tub formation assay was conducted at passages 2, 4, and 6 using PVECs to assess their endothelial behavior. For this assay, 150 µl of Matrigel solution (Corning, #356231) was added at a concentration of 3 mg/ml (diluted from the stock with EGM-2 medium without supplements) in an 8-well removable chamber slide (IBIDI, #80841) and incubated at 37°C for 30 min. After this period, the Matrigel matrix solidified at the bottom of the chamber, and cells were seeded on top of the matrix at a concentration of 2.5×10⁴ cells/250 µl. The cells were then incubated for 24 hours at 37°C, and images were captured at 4 hours and 24 hours to observe tubule formation.

To conduct this assay, all materials (including pipettes, tips, and chamber slides) were pre-cooled to 4°C before initiating the experiment. Matrigel aliquots were thawed at 4°C and handled rapidly to prevent premature solidification during manipulation.

### Immunofluorescence

PVECs were seeded into fibrinogen pre-coated removable 8-well chamber slides (IBIDI, #80841) at a concentration of 10000 cells/ml to obtain a monolayer after 24 h. Cells were then fixed with 4% paraformaldehyde (Santa Cruz, #SC-281692) for 10 min at room temperature, rinsed with PBS and permeabilized with 0.1% triton X-100 (Sigma) for 5 min. Thereafter, cells were blocked for 30 min with 1% BSA in PBS and subsequently incubated with primary antibodies against eNOS (1:100, BD Bioscience, #610297) and Von Willebrand factor (1:100, Dako, #A0082) overnight at 4°C. Incubation with secondary antibodies conjugated with Alexa Fluor 488/555 (1:300, Invitrogen) was performed at room temperature for 1 h and washed with PBS along with DAPI (3 ng/ml, ThermoFisher, #D1306). Preparations were then mounted using Fluoromount-G (SouthernBiotech, #0100-01) and dried overnight. Imaging was done using an Olympus BX51 fluorescent microscope.

### Immortalization of human portal vein endothelial cells

Passage 1 PVECs were seeded in a 24-well plate at a concentration of 1×10⁴ cells/ml. When cells reached 70% confluency, they were transduced with 1 µl/ml of lentiviral particles expressing the SV40 large T-antigen and containing the GFP-puromycin fusion dual marker (Amsbio, #LVP016-GP-PBS) according to manufacturer's instructions. Seventy-two hours after retroviral transduction, transduced cells were selected using EGM-2 medium supplemented with puromycin at 1,5 µg/ml. Additionally, positive selection for GFP⁺ cells were confirmed by flow cytometry. Immortalized cells could be expanded between 25-40 passages, while same patient non-transduced cells never grew beyond 10 passages. During all these passages cells were continuously characterized as previously described for primary PVECs.

### Tumorigenic soft agar assay

To prepare the lower layer, UltraPure^{™} Low Melting Point Agarose (Invitrogen, #16520050) was melt at 1% in EGM-2 medium without FBS in the microwave and cooled to 50°C in a water bath in an autoclaved glass bottle. One milliliter of soft agar was added to each well of a 6-well plate and allowed to solidify at room temperature for 15 min. To prepare the top layer, agarose was initially prepared at 0.8% in EGM-2 without FBS in the microwave and cooled at 40°C in a water bath. Prior to its use, FBS was added to the agar to obtain a 10% final concentration.

In parallel, 2.5 ml of complete EGM-2 medium containing 1×10⁶ cells/ml was prepared in a 15 ml falcon tube. Cells and agar 0.8% were then mixed in a ratio 1:1 by adding 2.5ml of the agar 0.8% on top of the cells, which diluted the agar to a final 0.4% concentration. The mixture was then gently shaked and 1.5ml of the mixture was finally added to each well (triplicates), seeding around 10000 cells/well. It was allowed to solidify 15 min at room temperature and, afterwards, 2 ml of EGM-2 complete media were added to each well. Cells were then left in the incubator (37°C) for 20 days to allow colony formation, adding 0.5ml of fresh medium every 4-5 days. Pictures were taken with Olympus IX71 microscope when colonies formed.

The experiment was conducted using epithelial cells from a pancreatic tumor (PANK) as positive control, non-immortalized PVECs as the negative control, and the immortalized cells to ascertain if they had acquired tumorigenic characteristics.

### Histologic and immunohistochemical studies

Portal veins and hepatic veins were fixed in either formalin or Paxgene, paraffin embedded and 4.5µm sections obtained. Hematoxylin and eosin (H&E) staining was performed for structural analysis. Vein wall composition was analyzed by Movat Pentachrome Stain Kit (Modified Russell-Movat, Abcam, #ab245884) to detect elastic fibers, collagen, mucin, fibrin, and muscle.

### Immunohistochemical staining

Portal vein slides were deparaffinized with xylene and ethanol and antigen retrieval performed with a citrate solution (pH 6). Endogenous peroxidases were inactivated with 3% hydroxide peroxide (Panreac, #1.410.761.211) incubation and slides blocked with antibody diluent with background reducing component at room temperature (Dako, #S3022). Immunostaining of CD68 (1:100, Dako, #M087601-2), CD3 (1:100, Dako, #A045229-2) were performed overnight at 4°C. Primary antibodies were visualized with Dako Real Envision Detection System- HRP (#K4001, #K4003), and slides were counterstained with hematoxylin (Sigma, #GHS232-1L).

### Histological analysis

Histological analysis was performed by an expert pathologist. The inflammatory infiltrates in the portal vein wall, Vimentin expression as well as the collagen, fibrin, mucin, muscle and elastic fibers content were evaluated using a score from 0 to 3 according to the following criteria: 0 = none; 1 = mild; 2 = moderate; 3 = severe. The assessment of Snail expression was performed as follows: total percentage of staining in the tunica intima and media of the analyzed portal vein wall.

### RNA extraction, sequencing and bioinformatic analysis

Passage 1 PVECs and HVECs from HV_CT (n = 2), P_CT (n = 3), P_CH (n = 12) and P_PVT (n = 5) were collected in RLT + β-mercaptoethanol and RNA was isolated with the RNeasy Micro kit (Qiagen) with on-column DNAsel treatment following manufacturer's instructions. RNA purity and quality was analyzed with Bioanalyzer and only samples with an RNA Integrity Number above 8 were used for subsequent steps.

Libraries were synthesized with the NEBNext Ultra II Directional RNA Library Prep Kit for Illumina (New England Biolabs) and sequencing was paired end (2x50 bp) on an Illumina NovaSeq 6000 sequencer, obtaining a minimum of 30 million read per sample. Sequence quality was checked with FastQC (version 0.11.9). Reads were pseudoaligned to the human genome (version GRCh38.p13) with the Kallisto aligner. Gene level TPM Counts calculation and differential expression analysis were performed with sleuth R package (version 0.30.1) using only protein-coding genes. Differentially expressed genes were (DEGs) were identified using the likelihood ratio test by pvalue < 0.05 and FOR < 0.2. Functional enrichment and drug repurposing analysis were performed with the fgsea R package (version 1.26.0), using the b-statistic as proxy for gene-level stats. Reactome, Kegg and Molecular Signatures (MSigDB, GO Biological Process ontology) databases were used as the source for functional annotation to uncover biological functions related to gene expression changes while the Drug Gene Interaction Database (DGldb) was used for information on drug-gene interactions. To agglomerate gene patterns along disease stages, Short Time-course Expression Miner (STEM) algorithm was used through on-line platform. To identify in an unbiased way the transcription factors predicted to be directly involved on transcriptomic changes we apply functional prediction of differentially expressed genes (DEG) by the use of CheA software from Enrichr platform.

### Simvastatin treatment

To evaluate the Simvastatin's potential effects on ameliorating EndMT on endothelial cells, PVECs (passages 4-6) were seeded in 6-well plates in triplicates (for RNA collection) and in 60mm dishes (for protein collection) at a concentration of 6×10⁴ cells/ml and grow for 24 h to obtain 70% confluency. Cells were then treated with 5 µM Simvastatin sodium salt (Sigma, #567022) for another 24 h and collected in either RLT or RIPA buffer for further analysis by means of qPCR or Western blotting, respectively.

### Quantitative real-time polymerase chain reaction

Total RNA was isolated by RNeasy Micro and Mini kit (Qiagen) as stated above. First-strand cDNA was synthesized using the High Capacity cDNA Reverse Transcription Kit (Applied, Thermo Fisher, #4368814) according to the manufacturer's instructions. Quantitative real-time PCR (qPCR) was performed using PowerUp^{™} sybr^{®} Green Master Mix (Applied Biosystems, #A25776) with technical duplicates using an AB1 Prism^{®} 7900 HT Cycler (Applied Biosystems). PCR cycle parameters were as follows: 95 °C for 10' followed by 40 cycles at 95 °C for 15" and 60 °C for 1'. Results were obtained with the Sequence Detection System 3.2 software (Applied Biosystems) and further analyzed by the 2^{-ΔΔCt} method. GAPDH was used as a loading control. Results are shown as fold-change.

Primer specific sequences are listed in Table 1 below:

**Table 1**

| **Gene** | **Primer name** | **Primer sequence** | **SEQ ID NO:** |
|---|---|---|---|
| GAPDH | GAPDH Fw | TCGGAGTCAACGGATTTGGT | 1 |
| | GAPDH Rv | TTCCCGTTCTCAGCCTTGAC | 2 |
| Cd31/Pecam1 | CD31 Fw | GAAAGCTGTCCCTGATGCCG | 3 |
| | CD31 Rv | ATGTCCTTCCAGGGATGTGC | 4 |
| NOS3 | NOS3 Fw | CGAGTGAAGGCGACAATCCT | 5 |
| | ENOS Rv | ACAGGACCCGGGGATCAAAA | 6 |
| CDH2 | CDH2 Fw | AGGCTTCTGGTGAAATCGCA | 7 |
| | CDH2 Rv | GCAGTTGCTAAACTTCACATTGAG | 8 |
| AIFM2 | AIFM2 Fw | CATGGTGAGGCAGGTCCAG | 9 |
| | AIFM2 Rv | GGCCACTTGGGAGTGAATGA | 10 |
| VCAM1 | VCAM1 Fw | TGGATAATGTTTGCAGCTTCTCAA | 11 |
| | VCAM1 Rv | AGATGTGGTCCCCTCATTCG | 12 |
| SERPINE1 | SERPINE1 Fw | ACCTCTGAGAACTTCAGGATGC | 13 |
| | SERPINE1 Rv | GGGTGAGAAAACCACGTTGC | 14 |
| RAC2 | RAC2 Fw | GGCCAAGGAGATTGACTCGG | 15 |
| | RAC2 Rv | GCCTCGTCGAACACGGTTT | 16 |
| SNAI1 | SNAI1 Fw | CGAGTGGTTCTTCTGCGCTA | 17 |
| | SNAI1 Rv | CTGCTGGAAGGTAAACTCTGGA | 18 |

### Results

### Portal vein wall suffers important vascular remodeling of its wall during liver damage

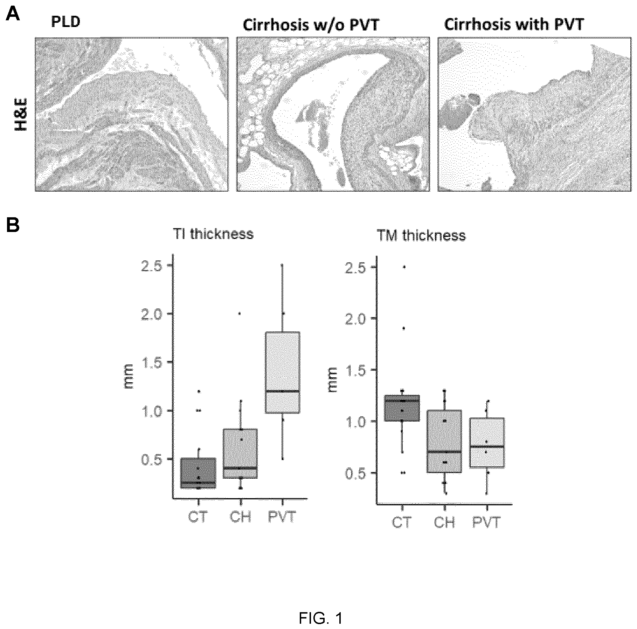

Portal vein (PV) wall undergoes significant changes in its composition during liver damage consistent of tunica intima thickening, fibrosis and tunica media degeneration (Figure 1).

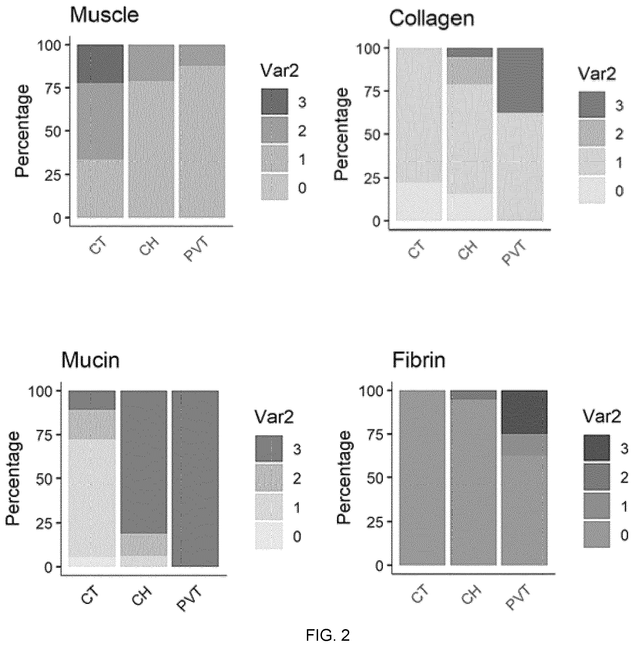

The subendothelial layer was identified as the primary driver of this thickening since it showed an enhanced secretory phenotype, evidenced by an increased mucin and extracellular matrix (ECM) deposition and reorganization (Figure 2). Histological changes were more pronounced in patients with portal vein thrombosis (PVT), suggesting a more advanced remodeling of PV wall.

### Characterization of PVECs

In the attempt to understand the role of endothelial cells from portal vein during PVT development and their specific contribution to the anatomical changes described above, the endothelium of human portal vein were isolated as described above for the first time to the best of the inventors' knowledge.

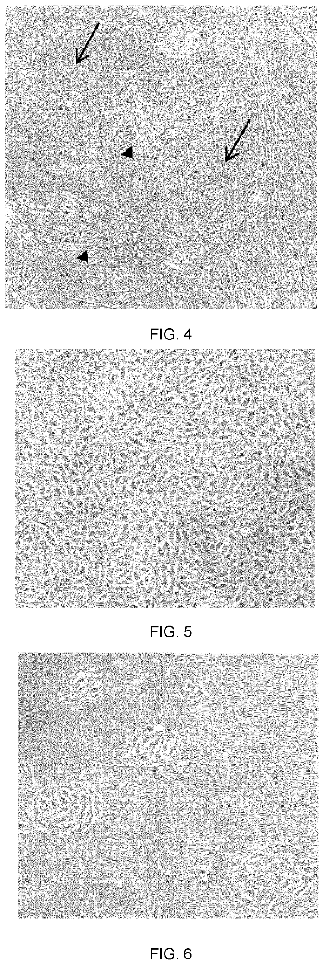

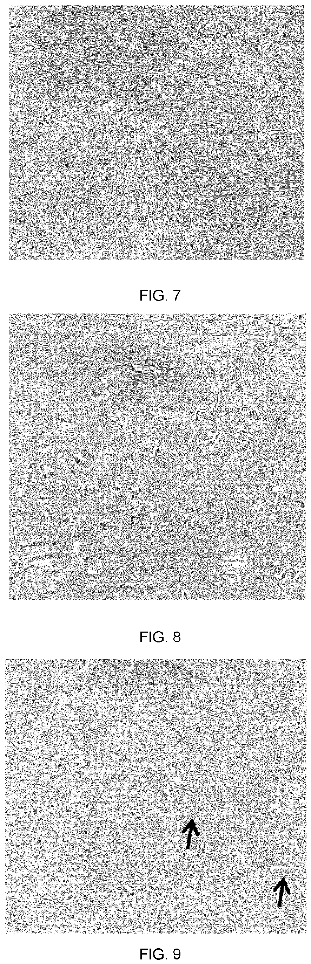

From the isolation of portal vein endothelial cells from a human portal vein piece of tissue as described in materials and methods above, not only the endothelial cells were obtained, but also isolated mesenchymal, muscular, or epithelial cells as shown in Figure 4. From the mix of cells the endothelial cells population were then purified selecting by CD31 plus CD144 positive markers. Once endothelial cells were purified, cell cultures of PVECS with a uniform morphology were obtained, forming uniform monolayers that proliferate normally as plaques of small clusters of cells that cluster tightly and were very adhesive between them (Figure 5 and Figure 6). Non-endothelial cells presented a clear differentiated phenotype (elongated and plump spindle shaped) and grew much faster than PVEC (Figure 7).

Between passage 6 and passage 9, PVECs started to gain a senescent phenotype (Figure 8 and Figure 9) with enlarged, flattened, multinucleated, and bigger morphologies. They also presented cytoplasmic granularities and they stopped growing.

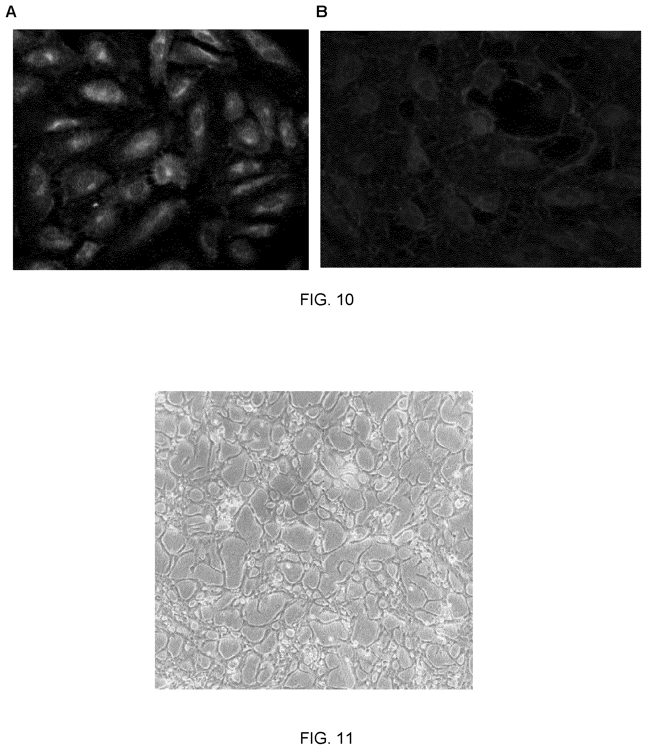

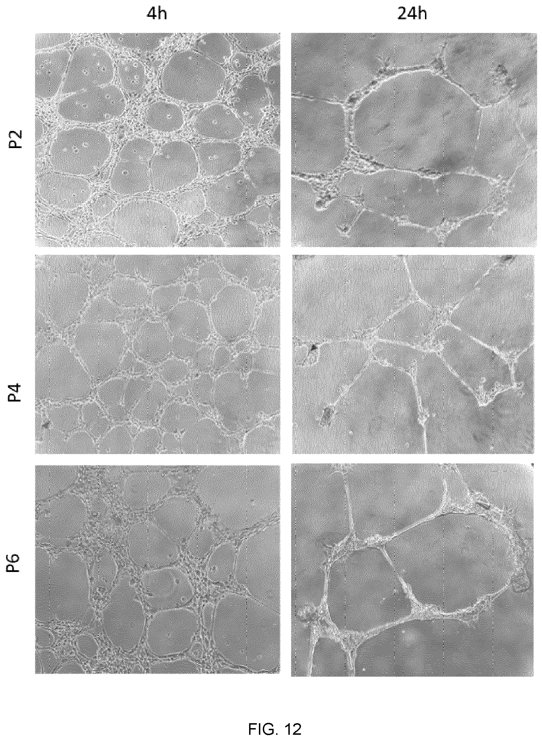

The isolated PVECs showed endothelial features through positive staining for eNOS and vWF (Figure 10) as well as endothelial behavior shown by their capacity to form tubes in a Matrigel matrix (Figure 11 and Figure 12).

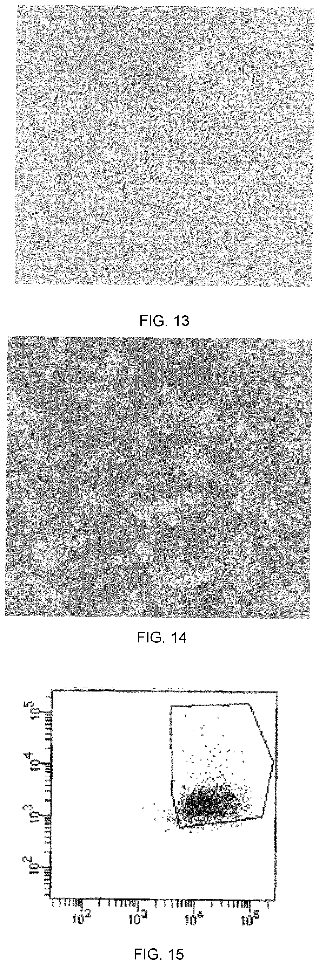

Moreover, the endothelial cell markers CD31 and VE-cadherin (CD144) was maintained among passages, showing PVECs did not suffer any modification in their endothelial phenotype when cultured (Figure 15).

All the above described characteristics were seen equally for all the PVECs isolated, independently of their patients' origin.

PVECs were then immortalized from the three different types of patients (PLN n=3, CH without PVT n=3 and CH with PVT n=4) by transducing passage 1 cells with lentiviral particles expressing the SV40 large T-antigen and containing the GFP-puromycin fusion dual marker. Cells demonstrated to be infected due to their resistance to Puromycin together with the GFP+ expression in flow cytometry.

When comparing their growth rates between the non-immortalized PVECS and the immortalized (IM) ones, it was observed a normal growing rate in the immortalized cells but they could grow until passage 25-40, which was significantly different from their homologues non-immortalized cells, which become senescent at passage 7-9 (Figure 8).

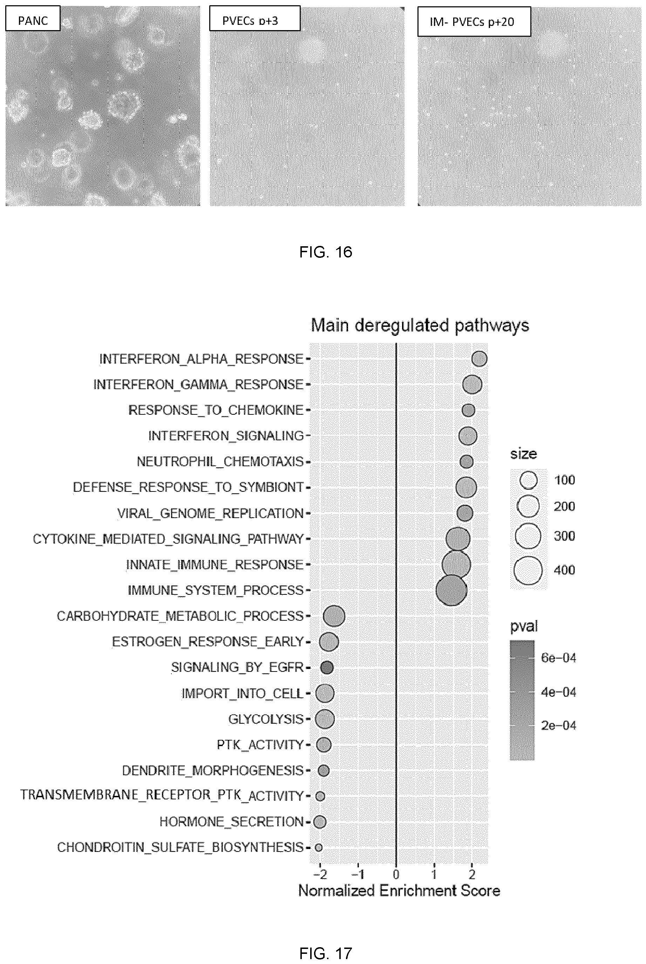

The IM cells conserved the normal phenotype and morphology (Figure 13), the expression of CD31 and CD144 endothelial markers among all passages (Figure 15) as well as their capacity of forming tubes (Figure 14). Moreover, they did not acquire tumorigenic phenotype since they did not form colonies in a soft agar assay (Figure 16).

### Portal vein endothelial cells undergo endothelial-to-mesenchymal transition during cirrhosis - Transcriptomic analysis of portal vein endothelial cells

A comprehensive analysis was carried out of the PVEC transcriptomic data from a series of cirrhotic patients without PVT (CH, n=12), cirrhotic patients with PVT (PVT, n=5) and a unique set of explants from patients with polycystic liver disease (PLD) undergoing liver transplantation (PLD, n=3), included as controls since they did not suffer from portal hypertension. Hepatic veins from PLD group (PLD-HV n=3) were included as external controls to be able to compare endothelial cells from the systemic venous territory versus PVECs, which belong to the splanchnic territory.

As a first step, to understand the effects of cirrhosis and the development of portal hypertension on the PVEC transcriptome, CT-PVEC with CH-PVEC were compared.

Results showed that endothelial cells from cirrhotic patients undergo significant up-regulation of a variety of immune related-pathways, primarily involving cytokine and chemokine signaling, along with immune cell migration (Fig. 17).

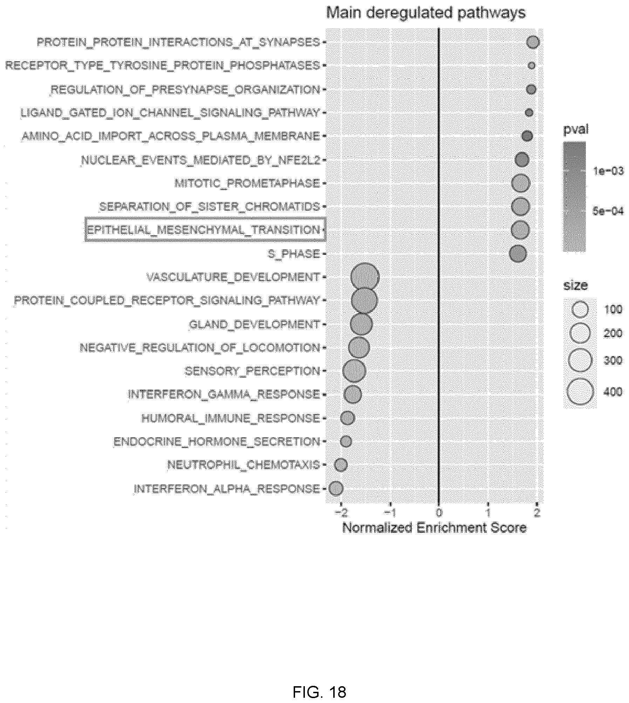

PVECs from cirrhotic patients without PVT and those with PVT were then compared, gene set enrichment analysis revealed up-regulation of genes related to endothelial to mesenchymal transition (EndMT) pathway as one of the most significantly altered processes, together with an increase in ECM production and organization (Fig 18). Immune regulation-related pathways were enriched among down-regulated genes as well as GTPases, suggesting CH-PVT-PVECs displayed a more secretory phenotype and increased permeability of the endothelial barrier (Fig 18).

Overall, these changes suggest that EndMT could be the driving mechanism behind intimal hyperplasia and TI proliferation.

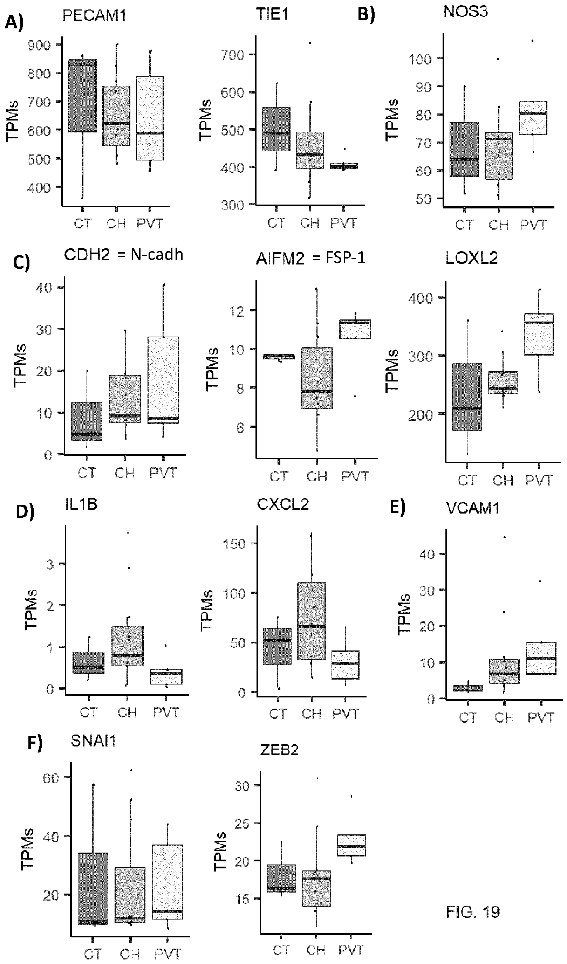

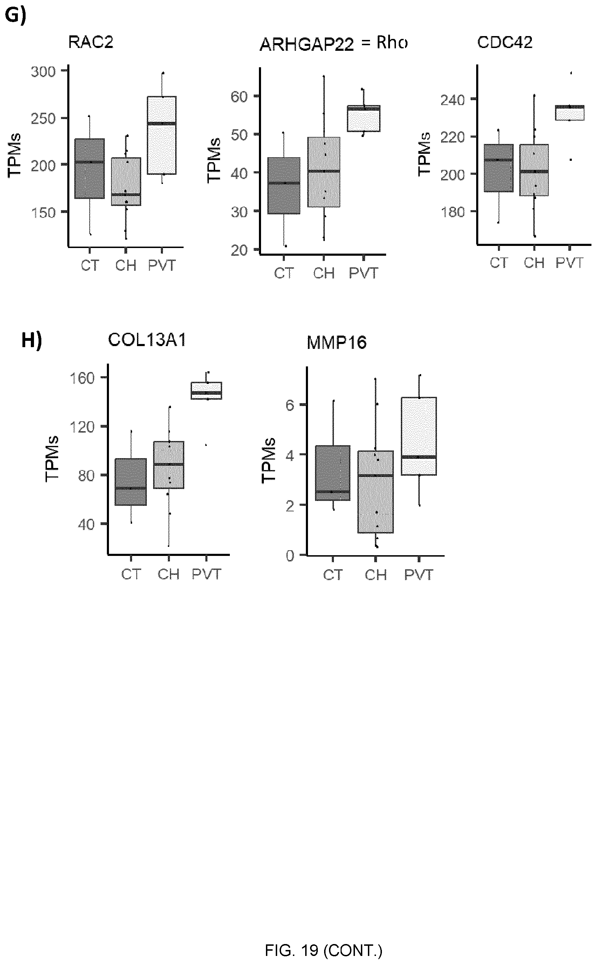

To delve deeper into the EndMT mechanism occurring in PVECs, the expression levels of the EndMT's hallmark genes was analyzed in the four different groups. Hallmarks of EndMT are known to include:
- the loss of EC markers and function (PECAM1 and TIE1) (Fig. 19 a,b)
- accompanied by the gain in mesenchymal cell markers (N-cadh and FSP-1) and functions (LOXL2) (Fig. 19c)
- deregulation of inflammation (IL1b and CXCL2) (Fig. 19d)
- increased expression of adhesion molecules (VCAM1) (Fig. 19e)
- increased expression of TF governing EndMT (SNAI1 and ZEB2) (Fig. 19f)
- dramatic cytoskeletal reorganization (triggered by GTPases Rho and Rac) (Fig. 19g)
- ECM remodeling (COL13A1 and MMP16) (Fig 19h)

The expression of all these EndMT's hallmark genes were markedly altered in PVT and, importantly, analysis of these results suggested changes seem to display a progressive pattern as disease advances.

### EndMT occurs as a progressive pathological mechanism in portal vein

To explore the progressive nature of the observations described above, the data was analyzed with the Short Time Expression Miner (STEM) algorithm, which grouped genes by their time-correlated patterns of expression. The progression of the disease was considered as being: PLD-HV (systemic territory), PLD-PVEC (splanchnic territory), CH-PVEC (disease onset) and CH-PVT-PVEC as the final step of this progressive development of portal vein events.

The STEM algorithm identified 7 significant profiles of gene expressions across the 4 selected stages.

These profiles were grouped into 3 main clusters, reflecting consistently up- or down-regulated genes along disease progression on one hand, and compensatory gene expression changes due to the presence of PVT on the other. A detailed gene set enrichment analysis from these different clusters revealed a continuous up-regulation of genes related to ECM and GTPases (cluster 21) during disease progression, while cell-junction organization related pathways were enriched among down-regulated genes (cluster 4). Transient compensatory genes during PVT appearance were primarily related to immune pathways, but also GTPases, which could reflect a response to the altered immune profile and cytoskeletal reorganization (cluster 24).

### Immune-cell infiltration in the portal vein wall triggers EndMT via TGFβ/SMAD in portal vein

It was next interrogated which transcription factors could be driving the coordinated gene expression changes observed during PVECs remodeling. Specifically, the differentially expressed genes (DEGs) between CH-PVECS and CH-PVT-PVECs were searched and used the ChEA software to compute over-representation of transcription factor targets. The analysis uncovered an increase in predicted upstream activity of mainly SMAD2 and SMAD3. SMAD transcription factors are the principal downstream effectors of TGF-β signaling, and are able to activate other transcription factors like Snail, which is one of the most important TF in the onset of EndMT.

In agreement with the transcriptomic analysis, histological examination of PV revealed a significant increase in immune cell infiltration characterized by the presence of CD68-positive macrophages and CD3-positive leukocytes in the intimal layer of portal veins form cirrhotic patients compared to PLD, which was even more pronounced in the portal vein of cirrhotic patients with PVT (Fig. 10). All these myeloid cells present in the portal vein wall are known to be secreting TGFβ, which could be the main driver of EndMT.

Altogether, these findings suggest that the EndMT occurring in the PV, that ultimately may lead to PVT development, could be the result of increased immune cell infiltration into the vein wall, with the subsequent increased secretion of TGFβ and endothelial activation of the SMAD-Snail pathway.

### Drug repositioning analysis reveals statins as potential disease modifier in PVT

It was next explored drug repurposing for EndMT prevention or reversion in the context of PVT. For this purpose, a gene set enrichment analysis was performed on the DEGs between CH-PVECS and CH-PVT-PVECs with the Drug Gene Interaction Database (DGldb), which contains the curated association between 1105 drugs and genes.

From this analysis, several compounds emerged as potential drugs for targeting the altered pathways in our PVECs from PVT patients. Among those, statins seemed to be the more interesting drugs.

The majority of studies exploring the use of statins in liver disease have focused on Simvastatin, a widely used statin with a good safety record in cases of compensated cirrhosis. In clinical applications, simvastatin has shown promise in improving hepatic microcirculation and reducing portal hypertension in both compensated and decompensated cirrhosis. Moreover, simvastatin offers additional potential advantages, including immune cell function modulation, effects on endothelial immunotolerance, potential influence on gut microbiota, and anticoagulant effects.

Furthermore, statins have shown the potential to improve EndMT in several studies by improving EC phenotype and reducing contractility of the cells. Also, statins have been described to reduce neointimal inflammation (macrophage infiltration) in a rabbit model of atherosclerosis and impedes small GTPases, which are the same mechanisms acting in PV wall remodeling in cirrhosis.

### Simvastatin can ameliorate PVEC EndMT hallmarks

It was then sought to determine whether EndMT could be reversed or ameliorated. To address these questions, PVECs from CT (n=3), CH (n=7) and CH-PVT (n=6) were treated *in vitro* with Simvastatin for 24 h and studied its effect on mesenchymal markers and EndMT promoters, including TFs and all the molecules involved in EndMT initiation or progression described previously.

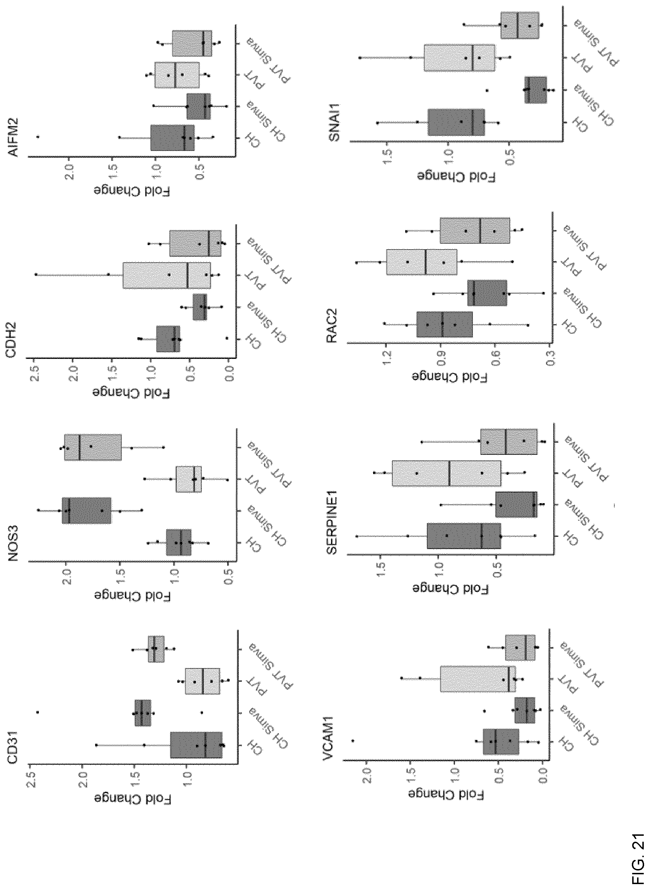

Results demonstrated, for the first time, that Simvastatin can ameliorate the EndMT occurring in PVEC (Fig. 21). Simvastatin was capable to increase endothelial markers, reduce the expression of mesenchymal markers as well as VCAM1 and, importantly, it reduced the expression of transcription factor Snail1. Furthermore, Simvastatin exhibited a role in decreasing GTPase (Rac2) and Serpine1 expression.

Its role in decreasing phosphor-SMAD2 levels may be the key in preventing EndMT initiation/progression. Simvastatin might be acting as an inhibitor of Smad2/3 activation in PVECs, potentially offering a strategy for preventing PVT formation.

### Simvastatin treatment in cirrhotic patients

A cohort of cirrhotic patients were prospectively evaluated until the development of portal thrombosis. Some patients were taking statins to treat hypercholesterolemia and hence the difference between these patients and patients not taking statins were analyzed.

As shown in Table 2, it was observed that PVT development was more frequent in patients who did not take statins:

**Table 2**

| Baseline variables | PVT | NO PVT |
|---|---|---|
| | N = 30 | N = 339 |
| Age, years - median (IQR) | 58.5 (51.5 - 63.8) | 59 (52 - 67) |
| BMI - median (IQR) | 26.4 (24.8 - 30.5) | 26.9 (24.7 - 29.7) |
| Gender Female - number of patients (%) | 13 (43.3%) | 152 (41.2%) |
| Etiology of liver disease - number of patients (%) HCV | | |
| HBV-HBV/HDV | 8 (26.7%) | 173 (51%) |
| Alcohol | 2 (6.7%) | 16 (4.7%) |
| Cholestatic + Autoimmune | 8 (26.7%) | 90 (26.5%) |
| NASH | 2 (6.7%) | 11 (3.2%) |
| Other | 3 (10%) | 10 (2.9%) |
| | 7 (23.3%) | 39 (11.5%) |
| Child-Pugh score - number (%) | | |
| A | 16 (53.3%) | 250 (73.7%) |
| B | 13 (43.3%) | 67 (19.8%) |
| C | 1 (3.3%) | 22 (6.5%) |
| MELD - median (IQR) | 11 (9.3-13.8) | 9 (7.3 -11) |
| HCC (number of patients (%) | 0 | 0 |
| Varices - number of patients (%) | 24 (80%) | 198 (58.4%) |
| Ascites (number of patients (%) | 16 (53.3%) | 125 (36.9%) |
| Controlled with diuretics | 7 (25.9%) | 44 (13.4%) |
| Moderate | 8 (29.6%) | 62 (18.8%) |
| Severe | 1 (3.3%) | 19 (5.8%) |
| Encephalopathy (number of patients (%) | 5 (16.7%) | 46 (13.6%) |
| Liver Stiffness, KPa - median (IQR) * *available in* 34% *and 68%* | 24.7 (13.4 - 46) | 23.5 (16.9 - 35.8) |
| HVPG, mmHg - median (IQR) * *available in* 59% *and 34%* | 17.8 (12.9 - 21) | 15 (12 -18.5) |
| Splenomegaly - number of patients (%) * *available in 74% of patients* | 15 (71.4%) | 101 (40.1%) |
| Portal flow velocity, cm/sec - mean (± DS) | 15.1 (± 7.6) | 17.9 (± 5.4) |
| Platelets, × 109/L - median (IQR) | 75 (60.5-90) | 106 (75 - 145.5) |
| INR - median (IQR) | 1.3 (1.2 -1.4) | 1.2 (1.1 - 1.3) |
| Creatinine, mg/dl - median (IQR) | 0.8 (0.7 - 0.9) | 0.82 (0.71 - 0.94) |
| Sodium, mEq/L - median (IQR) | 141 (140 - 141.8) | 141 (139 - 142) |
| Bilirubin, mg/dl - median (IQR) | 1.5 (1 - 2.6) | 1.1 (0.8 - 1.6) |
| Albumin, g/L - median (IQR) | 36 (31.3 - 39) | 38 (35 - 42) |
| Statins - number of patients (%) | 1 (3.3 %) | 16 (4.7%) |

### Citation List

WO1997032972
de Franchis, Roberto et al. "Baveno VII - Renewing consensus in portal hypertension." Journal of hepatology vol. 76,4 (2022): 959-974

## Claims

1. An isolated human portal vein endothelial cell (PVEC).

2. The isolated human portal vein endothelial cell (PVEC) according to claim 1, which is originated from human adult liver.

3. The isolated human portal vein endothelial cell (PVEC) according to claim 2, wherein the human adult liver is selected from human adult liver without cirrhosis and/or portal hypertension, and cirrhotic human adult liver; particularly the human adult liver is cirrhotic human adult liver with portal vein thrombosis (PVT).

4. The isolated human portal vein endothelial cell (PVEC) according to any one of claims 1-3, which expresses the cell surface markers CD31 (cluster of differentiation 31) and CD144 (cluster of differentiation 144).

5. The isolated human portal vein endothelial cell (PVEC) according to any one of claims 1-4, which is an immortalized cell.

6. The isolated human portal vein endothelial cell (PVEC) according to claim 5, wherein the cell comprises an SV40 antigen; particularly an SV40 large T-antigen.

7. An isolated cell population comprising an isolated human portal vein endothelial cell (PVEC) as defined in any one of claims 1-6.

8. A composition comprising an isolated human portal vein endothelial cell (PVEC) as defined in any one of claims 1-6 or an isolated cell population according to claim 7.

9. A microfluidic device comprising an isolated human portal vein endothelial cell (PVEC) as defined in any one of claims 1-6, an isolated cell population as defined in claim 7, or a composition as defined in claim 8.

10. The microfluidic device according to claim 9, which comprises a hydrogel matrix; particularly wherein the hydrogel matrix comprises one or more internal spaces coated with the isolated human portal vein endothelial cell (PVEC) or the isolated cell population.

11. *In vitro* use of an isolated human portal vein endothelial cell (PVEC) as defined in any one of claims 1-6, an isolated cell population as defined in claim 7, a composition as defined in claim 8, or a microfluidic device as defined in claim 9 or 10, for identifying a compound useful in the treatment and/or prevention of a hepatic disease or disorder; particularly a compound useful in the treatment and/or prevention of portal vein thrombosis (PVT).

12. An *in vitro* method for identifying a compound useful in the treatment and/or prevention of a hepatic disease or disorder, particularly portal vein thrombosis (PVT), the method comprising the step of contacting a compound with an isolated human portal vein endothelial cell (PVEC) as defined in any one of claims 1-6, a cell population as defined in claim 7, a composition as defined in claim 8, or a microfluidic device as defined in claim 9 or 10.

13. The *in vitro* use according to claim 11 or the *in vitro* method according to claim 12, wherein the compound is determined to be useful in the treatment and/or prevention of a hepatic disease or disorder, particularly useful in the treatment and/or prevention of portal vein thrombosis (PVT), when the compound reduces, prevents, or reverts the endothelial-to-mesenchymal transition (EndMT) of the isolated human PVEC.

14. An *in vitro* method for preparing an isolated human portal vein endothelial cell (PVEC), the method comprising the steps of:
a) dissociating the inner wall of a human portal vein or a part thereof to generate a cell suspension;
b) plating the cell suspension onto a substrate which allows adherence of cells thereto; and
c) culturing the plated cells under suitable conditions.

15. The isolated human portal vein endothelial cell (PVEC) according to any one of claims 1-6, which is obtainable by the method as defined in claim 14.
